# EUROPEAN PATENT APPLICATION

(11) **EP 4 563 166 A1**
(43) Date of publication of application: **04.06.2025**
(21) Application number: 24460004.5
(22) Date of filing: 18.01.2024
(51) Int. Cl.: A61L 2/08, A61L 2/10, A61L 2/20, B65B 55/02, B65B 55/08

(54) **CONTAINER, IN PARTICULAR PALLET BOX, DISINFECTION SYSTEM**

(30) Priority: 30.11.2023 PL 44690823
(71) Applicant: KMK Agro D. Kazmierczak C. Madry M. Kazmierczak Sp. J., 63-000 Sroda Wielkopolska (PL)
(72) Inventor: Kazmierczak, Dariusz, 63-012 Dominowo (PL); Krajewski, Adrian, 63-000 Sroda Wielkopolska (PL)
(74) Representative: Urbanska-Luczak, Barbara

(57) **Abstract**

The subject of the invention is a container, in particular pallet box, disinfection system, which is to be used in particular in process lines where such products as vegetables or fruit are packed, in particular potatoes.

A system according to the invention is characterized in that the dome is composed of a dome bottom (1) and a dome cover (2), advantageously cuboid, and the dome cover (2) is installed in a sliding manner in relation to the dome bottom (1), whereas the dome cover (2) consists of an upper base and side walls permanently and tightly connected to it, whereas to the dome bottom (1) and/or to at least one of the walls of the dome cover (2) conduits are attached, which supply ozone from an ozone generator, and a system for distributing of ozone (14) and a source of bactericidal light and/or a source of virucidal light (15), advantageously an UV-C lamp, are installed inside the dome, whereas advantageously the ozone distribution system (14) consists of at least one conduit, in the zone of which is installed at least one fan (16).

## Description

The subject of the invention is a container, in particular pallet box, disinfection system, which is to be used in particular in process lines where such products as vegetables or fruit are packed, in particular potatoes. Systems for washing and disinfection of packaging, in particular containers, constitute an area of technology.

The state of the art comprises many systems for the washing of packaging and containers, where usually detergents and various types of chemicals with a negative environmental impact are used.

For example, the European patent EP 2709931 demonstrates a method for disinfecting and cleaning a garbage container, which uses a cleaning or disinfection device that is placed inside a housing and is configured to supply a cleaning or disinfection fluid to the interior of the container after the container is emptied by tilting to the unloading position. A solution according to the invention is characterised by a stage of measuring a dose of liquid inside the container when it is tilted to the unloading position and a stage of spreading of the dose of liquid inside the container, after the container has been returned to its normal position of use, where the state of measuring a dose of liquid and the stage of spreading of the dose of liquid occur automatically one after another when the container is, respectively, in its unloading position and normal use position.

Moreover, washing processes are time-consuming, sometimes require the use of closed-circuit washing medium, making the optimalisation of process line much more difficult, complicating its design and sometimes even preventing the automation of packaging processes with a disinfection system built into the process line.

The solution according to the invention eliminates the drawbacks and disadvantages resulting from the use of solutions from the state of the art.

The essence of the invention, which is a container, in particular pallet box, disinfection system having a form of a closing dome, containing a dome bottom and a dome cover, where the dome cover is installed in a sliding manner in relation to the dome bottom, consists of the dome being composed of a dome bottom and a dome cover, advantageously cuboid, and the dome cover is installed in a sliding manner in relation to the dome bottom, whereas the dome cover consists of an upper base and side walls permanently and tightly connected to it, whereas to the dome bottom and/or to at least one of the walls of the dome cover conduits are attached, which supply ozone from an ozone generator, and a system for distributing of ozone and a source of bactericidal light and/or a source of virucidal light, advantageously an UV-C lamp are installed inside the dome, whereas advantageously the ozone distribution system consists of at least one conduit, in the zone of which is installed at least one fan.

It is advantageous when the dome bottom consists of a lower base of the dome bottom and side walls permanently and tightly connected to it.

It is also advantageous when in any of the side walls of the dome bottom and/or the dome cover there are cut-outs, which enable installation of guide rails or other elements of the transport conveyor which feeds the disinfected container, advantageously a pallet box, into the dome.

It is particularly advantageous, when to the upper base of the dome cover an insert is attached, advantageously cuboid, which decreases the volume of the disinfection chamber within the dome walls, in which the disinfected container, in particular a pallet box, is placed.

Additionally, it is advantageous when the source of the bactericidal light and/or a source of virucidal light, advantageously an UV-C lamp are attached to any of the walls of the insert.

It is also advantageous when between the dome bottom and the dome cover a dome seal is installed, and moreover it is advantageous when elements of a seal pressure mechanism are attached to the dome bottom and/or to the side walls of the dome cover.

It is also advantageous when the dome, consisting of a dome bottom and a dome cover is attached to the system frame in such a manner that the dome bottom is permanently connected to the frame, whereas the dome cover is attached to the frame in a sliding manner, whereas the system according to the invention has a dome cover raising drive which is connected to the dome cover by a raising system, advantageously with a tensioning system, whereas most advantageously the dome cover raising system constitutes an electric motor, and the dome raising system constitutes a belt-based raising system. It is also advantageous when guides, advantageously guide rods, are attached to the vertical elements of the frame, while to the external elements of the dome cover guide rollers are attached. It is also advantageous, when to the vertical elements of the frame at least one position sensor, advantageously two positions sensors, are attached, with most advantageously a dome cover lower position sensor being attached in the dome bottom zone, and a dome cover upper position sensor being attached above it.

Moreover, it is advantageous when the system contains at least one medium feeding guide.

The use of the solution according to the invention, the essence of which was presented above, enables achieve the following technical and utility effects:
- the elimination of the need to use detergents and chemical agents, as an effect improvement of the environmental impact of the process,
- the reduction of process time,
- the automation of packaging processes and optimalisation of process line due to elimination of the need to use closed circuits of the washing agent.

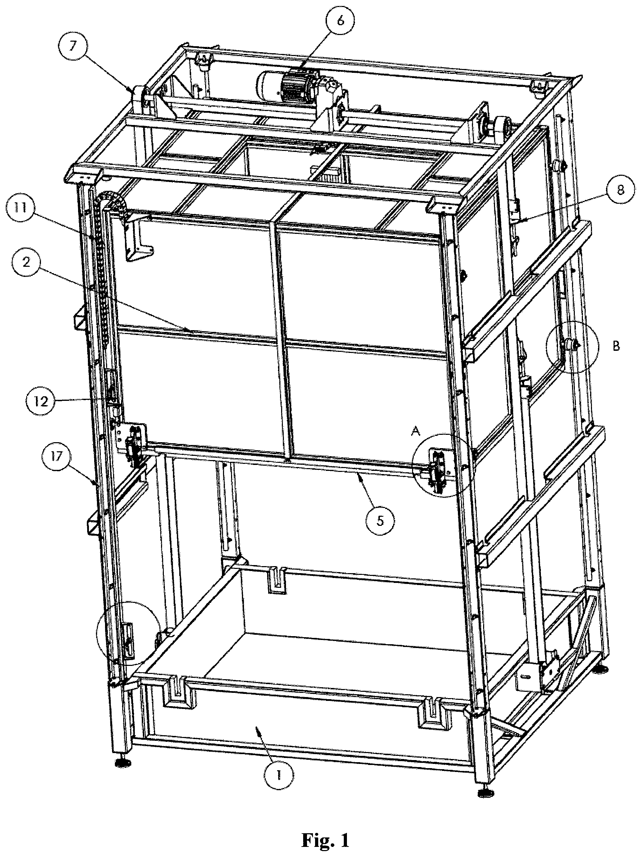

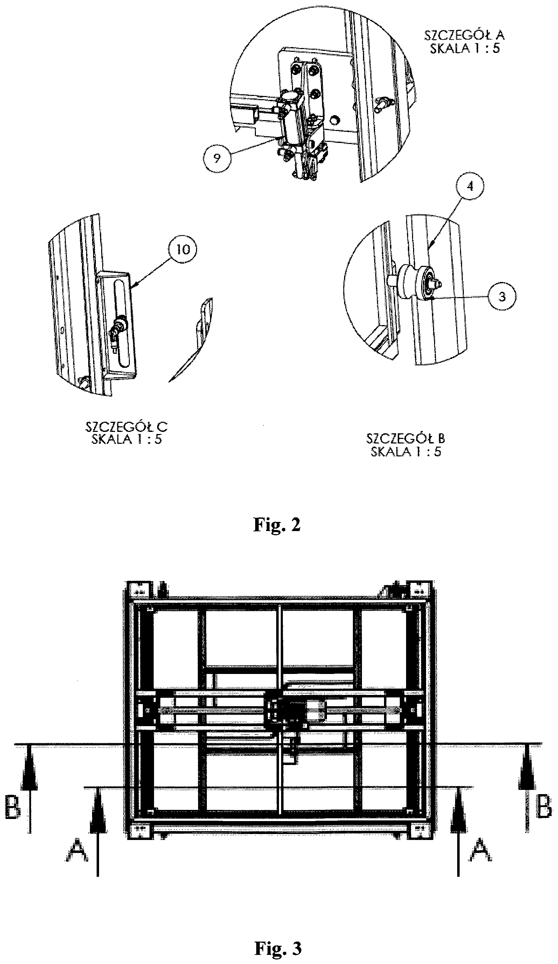

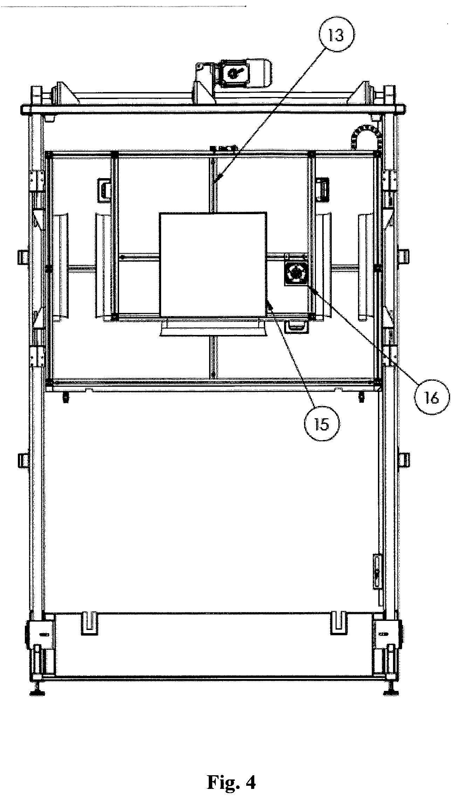

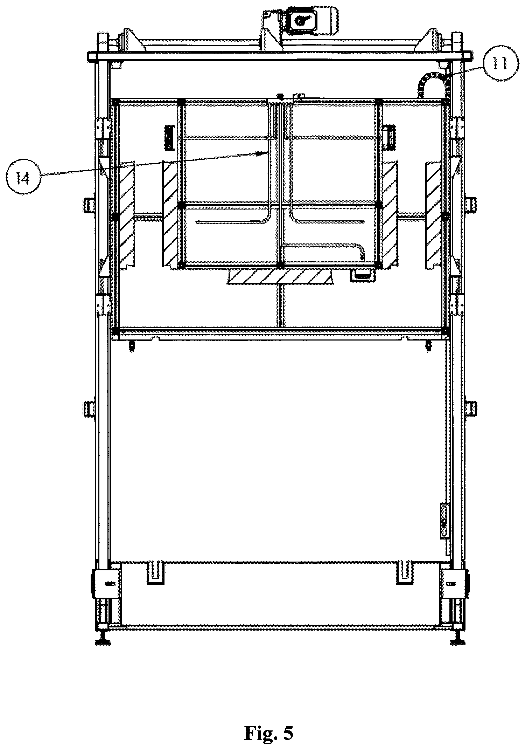

A solution according to the invention in an example, but not limiting implementation was presented on the figure.

A container disinfection system in an example implementation is used in particular to limit viruses and bacteria which may be present in the pallet boxes, which are filled with potatoes.

In the example implementation the system has the form of a cuboid dome, consisting of the dome bottom 1 and the dome cover 2. The dome bottom 1 consists of the bottom base and side walls permanently and tightly connected to it, whereas the dome cover 2 consists of an upper base and side walls permanently and tightly connected to it, whereas in two opposite side walls of the dome bottom 1 there are cut-outs, which enable installation of guide rails or other elements of the transport conveyor, advantageously guide rails of the transport conveyor, which feeds the disinfected pallet box into the dome. Between the dome bottom 1 and the dome cover 2 a dome seal 5 is attached. Additionally, to the side walls of the dome bottom 1 and to the side walls of the dome cover 2 elements of a mechanism 9 which provides pressure to the seal 5 are attached.

To one of the walls of the dome cover 2 using appropriate connectors known from prior art a conduit is attached which supplies ozone from an ozone generator to the inside of the dome, with the ozone generator constituting prior art and not being the subject of the invention, and thus it was not presented on the figure. Inside the dome a system for distributing of ozone 14 and a source of bactericidal and virucidal light 15 are installed, which in the example implementation is an UV-C lamp, whereas in the zone of conduits of the system for distributing ozone inside the chamber a fan 16 is installed.

The dome bottom 1 and the dome cover 2 are installed in the frame 17, with the dome cover 2 being installed in a sliding manner in relation to the dome bottom 1, where the dome bottom 1 is permanently attached to the frame 17. To the upper base of the dome cover 2 on its inner side a cuboid insert 13 is installed, which decreases the volume of the disinfection chamber within the dome walls, in which the disinfected pallet box is placed. UV-C lamps are attached to individual walls of the insert 13.

In the system according to the invention in the example implementation the dome, consisting of the dome bottom 1 and the dome cover 2 is attached to the disinfection system frame 17, in such a manner that the dome bottom 1 is permanently attached to the frame 17, whereas the dome cover 2 is attached in a sliding manner to the frame 17, with the system according to the invention having a drive 6 for raising the dome cover 2, which constitutes an electric motor connected to the dome cover 2 through a belt-based raising system 7 with a tensioning system 8. To the vertical elements of the frame 17 guide rods 4 are attached, and to external walls of the dome cover 2 guide rollers 3 are attached, which move on guide rods 4. To the vertical elements of the frame 17 moreover two position sensors are attached, where in the zone of the dome bottom 1 a dome cover 2 lower position sensor 10 is attached, and above this sensor a dome cover 2 upper position sensor 10 is attached.

Moreover, a medium feeding guide 11 is attached to the frame 17.

### List of designations

1. Dome bottom
2. Dome cover
3. Dome cover guide rollers in the frame
4. Dome cover guides in the frame
5. Dome seal
6. Dome cover raising drive
7. Dome cover raising system
8. Raising mechanism tensioner system
9. Dome cover to dome bottom pressure mechanism
10. Dome cover lower position sensor
11.Medium feeding guide
12.Dome cover upper position sensor
13. Insert
14.Dome ozone distribution system
15. Source of bactericidal light and/or a source of virucidal light
16.Fan
17.Frame

## Claims

1. A container, in particular pallet box, disinfection system having a form of a closing dome, containing a dome bottom and a dome cover, where the dome cover is installed in a sliding manner in relation to the dome bottom, **characterized in that** the dome is composed of a dome bottom (1) and a dome cover (2), advantageously cuboid, and the dome cover (2) is installed in a sliding manner in relation to the dome bottom (1), whereas the dome cover (2) consists of an upper base and side walls permanently and tightly connected to it, whereas to the dome bottom (1) and/or to at least one of the walls of the dome cover (2) conduits are attached, which supply ozone from an ozone generator, and a system for distributing of ozone (14) and a source of bactericidal light and/or a source of virucidal light (15), advantageously an UV-C lamp are installed inside the dome, whereas advantageously the ozone distribution system (14) consists of at least one conduit, in the zone of which is installed at least one fan (16).

2. A system in accordance with claim 1 **characterized in that** the dome bottom (1) consists of a lower base of the dome bottom and side walls permanently and tightly connected to it.

3. A system in accordance with claim 1 or claim 2 **characterized in that** the in any of the side walls of the dome bottom (1) and/or the dome cover (2) there are cut-outs, which enable installation of guide rails or other elements of the transport conveyor which feeds the disinfected container, advantageously a pallet box, into the dome.

4. A system in accordance with claim 1 **characterized in that** the to the upper base of the dome cover (2) an insert (13) is attached, advantageously cuboid, which decreases the volume of the disinfection chamber within the dome walls, in which the disinfected container, in particular a pallet box, is placed.

5. A system in accordance with claim 1 or claim 4 **characterized in that** the source of the bactericidal light and/or a source of virucidal light, advantageously an UV-C lamp are attached to any of the walls of the insert (13).

6. A system in accordance with claim 1 or claim 4 **characterized in that** between the dome bottom (1) and the dome cover (2) a dome seal (5) is attached.

7. A system in accordance with claim 1 or claim 6 **characterized in that** to the side walls of the dome bottom (1) and to the side walls of the dome cover (2) elements of a mechanism (9) which provides pressure to the seal (5) are attached.

8. A system in accordance with any of the claims, from claim 1 to claim 7, **characterized in that** the dome, consisting of the dome bottom (1) and the dome cover (2) is attached to the disinfection system frame (17), in such a manner that the dome bottom (1) is permanently attached to the frame (17), whereas the dome cover (2) is attached in a sliding manner to the frame (17), with the system according to the invention having a drive (6) for raising the dome cover (2) connected to the dome cover (2) through a raising system (7), advantageously with a tensioning system (8).

9. A system in accordance with claim 8 **characterized in that** the dome cover (2) raising drive (6) is an electric motor.

10. A system in accordance with claim 8 or claim 9 **characterized in that** the dome cover (2) raising system (7) consists of a belt-based raising system.

11. A system in accordance with claim 8 or claim 10 **characterized in that** guides (4), advantageously guide rods, are attached to the vertical elements of the frame (17), while to the external elements of the dome cover (2) guide rollers (3) are attached.

12. A system in accordance with claim 8 or claim 10 **characterized in that** to the vertical elements of the frame (17) at least one position sensor, advantageously two positions sensors, are attached, with most advantageously a dome cover (2) lower position sensor (10) being attached in the dome bottom (1) zone, and a dome cover (2) upper position sensor (10) being attached above it.

13. A system in accordance with any of the claims, from claim 1 to claim 12, **characterized in that** it possesses at least one medium feeding guide (11).
